(19) Europäisches Patentamt / European Patent Office / Office européen des brevets

(11) **EP 3 896 106 A1**

(12) **EUROPEAN PATENT APPLICATION**
published in accordance with Art. 153(4) EPC

(43) Date of publication:
20.10.2021 Bulletin 2021/42

(21) Application number: 19895551.0

(22) Date of filing: 12.12.2019

(51) Int Cl.:
C08F 265/02 (2006.01)    C08J 3/12 (2006.01)
C08J 3/24 (2006.01)    C08L 101/12 (2006.01)
C08F 2/44 (2006.01)    A61F 13/53 (2006.01)
B01J 20/26 (2006.01)    B01J 20/28 (2006.01)

(86) International application number:
PCT/JP2019/048799

(87) International publication number:
WO 2020/122204 (18.06.2020 Gazette 2020/25)

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
KH MA MD TN

(30) Priority: 12.12.2018 JP 2018232726
22.03.2019 JP 2019055121

(71) Applicant: Sumitomo Seika Chemicals Co., Ltd.
Kako-gun, Hyogo 675-0145 (JP)

(72) Inventor: NISHIDA, Moe
Himeji-shi, Hyogo 672-8076 (JP)

(74) Representative: Nederlandsch Octrooibureau
P.O. Box 29720
2502 LS The Hague (NL)

(54) **WATER-ABSORBENT RESIN PARTICLES**

(57) Disclosed is the water-absorbent resin particles including a crosslinked polymer having a structural unit derived from an ethylenically unsaturated monomer including at least one compound selected from the group consisting of (meth)acrylic acid and a salt thereof, in which a proportion of (meth)acrylic acid and a salt thereof is 70 to 100 mol% with respect to a total amount of monomer units in the crosslinked polymer, and a dissolved content is 10% by mass or more and 40% by mass or less, and a dissolved content when the water-absorbent resin particles are pulverized so that a median particle size is 80 to 165 $\mu$m is 15% by mass or more and 40% by mass or less, where the dissolved contents are measured by a specific method.

*Fig.1*

**Description**

**Technical Field**

The present invention relates to water-absorbent resin particles.

**Background Art**

A water-absorbing resin is used in the field of sanitary products and the like, and specifically, it is used as a material for an absorbent contained in an absorbent article such as a diaper. For example, Patent Literature 1 discloses an absorbent article including a super absorbent polymer as a hygroscopic agent.

**Citation List**

**Patent Literature**

[Patent Literature 1] JP H6-218007 A

**Summary of Invention**

**Technical Problem**

Because an absorbent article such as a diaper comes into direct contact with the skin when it is used, if stickiness of water-absorbent resin particles contained in the absorbent article is severe after they absorb water, this results in skin discomfort. Therefore, these water-absorbent resin particles used in the absorbent article are required to have less stickiness after they absorb water.
An object of the present invention is to provide water-absorbent resin particles having less stickiness after they absorb water, and an absorbent and an absorbent article which are formed from the water-absorbent resin particles.

**Solution to Problem**

The inventors of the present invention have thought the cause of generation of stickiness after water-absorbent resin particles absorb water when they are used in an absorbent article is a dissolved content eluted from the water-absorbent resin particles after they absorb water, and have made an attempt to produce water-absorbent resin particles with a small amount of a dissolved content. However, even when the water-absorbent resin particles with a small amount of a dissolved content were produced, stickiness after they absorb water could not be sufficiently suppressed.
Thereafter, the inventors of the present invention have conducted diligent research and have found that pulverization of water-absorbent resin particles is the cause of stickiness after they absorb water. In other words, the water-absorbent resin particles are partly disrupted (pulverized) due to the impact generated in a process of manufacturing an absorbent article (for example, the impact generated by transfer of the water-absorbent resin particles in a pipe, blowing of them with a high-speed air stream when producing an absorbent, and the like). The inventors of the present invention have found that, as compared with water-absorbent resin particles before being pulverized, the pulverized water-absorbent resin particles are likely to increase a dissolved content after they absorb water, which is the main cause of stickiness in an absorbent article. Furthermore, they have found that the water-absorbent resin particles with a small amount of a dissolved content after they absorb water even when they are pulverized can suppress stickiness, and thereby have completed the present invention.
The present invention provides water-absorbent resin particles in which a dissolved content when the water-absorbent resin particles are pulverized so that a median particle size is 50 to 200 $\mu$m is 40% by mass or less.
In the water-absorbent resin particles, a median particle size is preferably 250 to 600 $\mu$m.
In the water-absorbent resin particles, a proportion of particles having a particle size of 300 $\mu$m or less is preferably 55% by mass or less with respect to a total amount of the water-absorbent resin particles.
In the water-absorbent resin particles, the dissolved content may be a value when the water-absorbent resin particles are pulverized so that a proportion of particles having a particle size of 300 $\mu$m or less are 70% by mass or more with respect to a total amount of the water-absorbent resin particles.
The water-absorbent resin particles may have a water retention capacity for a physiological saline solution of 20 to 70 g/g.
The present invention further provides an absorbent containing the water-absorbent resin particles.
The present invention still further provides an absorbent article including the absorbent.

The absorbent article may be a diaper.

**Advantageous Effects of Invention**

According to the present invention, water-absorbent resin particles having less stickiness after they absorb water, and an absorbent and an absorbent article, which are formed using the water-absorbent resin particles, are provided.

**Brief Description of Drawings**

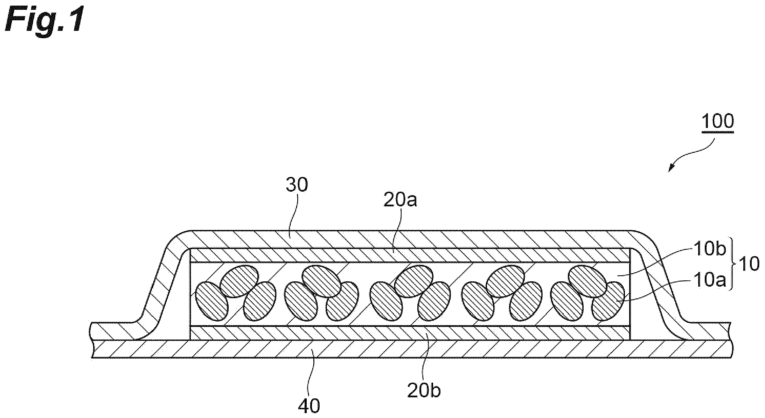

Fig. 1 is a cross-sectional view showing an example of an absorbent article.

**Description of Embodiments**

Hereinafter, suitable embodiments of the present invention will be described in detail. However, the present invention is not limited to the following embodiments.

In the present specification, "acrylic" and "methacrylic" are collectively referred to as "(meth)acrylic." "Acrylate" and "methacrylate" are also referred to as "(meth)acrylate." Regarding numerical value ranges described in a stepwise manner in the present specification, an upper limit value or a lower limit value of a numerical value range in a certain step can be arbitrarily combined with an upper limit value or a lower limit value of a numerical value range in another step. In a numerical value range described in the present specification, an upper limit value or a lower limit value of the numerical value range may be replaced with a value shown in examples. The term "water-soluble" means that a solubility of 5% by mass or more is exhibited in water at 25°C. For materials exemplified in the present specification, one kind may be used alone, or two or more kinds may be used in combination. In a case where there are a plurality of substances corresponding to each of components in a composition, a content of each of the components in the composition means a total amount of the plurality of substances present in the composition unless otherwise specified.

A median particle size of water-absorbent resin particles according to the present embodiment is preferably 250 to 600 $\mu$m. A median particle size of the water-absorbent resin particles according to the present embodiment may be, for example, 260 $\mu$m or more, 280 $\mu$m or more, or 300 $\mu$m or more. Furthermore, a median particle size of the water-absorbent resin particles may be, for example, 570 $\mu$m or less, 550 $\mu$m or less, or 500 $\mu$m or less.

In the water-absorbent resin particles according to the present embodiment, a proportion of particles having a particle size of 300 $\mu$m or less may be 55% by mass or less, 50% by mass or less, 45% by mass or less, 42% by mass or less, 40% by mass or less, 38% by mass or less, 35% by mass or less, 30% by mass or less, or 28% by mass or less, with respect to a total amount of the water-absorbent resin particles. A proportion of the particles having a particle size of 300 $\mu$m or less may be, for example, 0.5% by mass or more, 1% by mass or more, 3% by mass or more, 5% by mass or more, or 10% by mass or more, with respect to the total amount of the water-absorbent resin particles.

The water-absorbent resin particles according to the present embodiment can be made to have a desired particle size distribution at a timing obtained in a production method to be described later for example, but their particle size distribution may be set to a predetermined particle size distribution by further performing operations such as adjustment of a particle size through classification with a sieve.

The water-absorbent resin particles according to the present embodiment have a small amount of a dissolved content even when they are pulverized and their particle size is reduced. A dissolved content when they are pulverized so that a median particle size is 50 to 200 $\mu$m (dissolved content after pulverization) of the water-absorbent resin particles according to the present embodiment is 40% by mass or less. That is, a target for measurement of the dissolved content after pulverization is pulverized particles of the water-absorbent resin particles pulverized until a median particle size becomes 50 to 200 $\mu$m. Since the water-absorbent resin particles according to the present embodiment have a sufficiently low dissolved content after pulverization, stickiness after they absorb water is suppressed, and thereby discomfort when use can be reduced.

It is sufficient for a median particle size of the pulverized particles to be 50 $\mu$m or more, and it may be, for example, 70 $\mu$m or more or 80 $\mu$m or more. Furthermore, it is sufficient for a median particle size of the pulverized particles to be 200 $\mu$m or less, and it may be, for example, 180 $\mu$m or less, 170 $\mu$m or less, or 165 $\mu$m or less. In the pulverized particles, particles having a particle size of 300 $\mu$m or less may be, for example, 70% by mass or more, 75% by mass or more, 80% by mass or more, 85% by mass or more, 90% by mass or more, or 95% by mass or more, with respect to a total amount of the water-absorbent resin particles. In the pulverized particles, the particles having a particle size of 300 $\mu$m or less may be, for example, 100% by mass or less or 99% by mass or less with respect to the total amount of the water-absorbent resin particles. The above-described particle size and its proportion of the pulverized particles have been found by the inventors of the present invention as a range that is likely to be generated by pulverization when the water-absorbent resin particles are used for manufacturing an absorbent article.

A method of pulverizing the water-absorbent resin particles may be any method as long as the pulverized particles satisfy the above-mentioned conditions. The water-absorbent resin particles can be pulverized by using, for example, a pulverizer.

It is sufficient for a dissolved content after pulverization of the water-absorbent resin particles according to the present embodiment to be 40% by mass or less, and it may be, for example, 38% by mass or less, 35% by mass or less, 32% by mass or less, 30% by mass or less, or 28% by mass or less. It is desirable that a dissolved content after pulverization be as low as possible, but it may be, for example, 1% by mass or more, 10% by mass or more, 15% by mass or more, 18% by mass or more, 20% by mass or more, 23% by mass or more, or 25% by mass or more. In a case where a dissolved content after pulverization is within this range, stickiness after the water-absorbent resin particles absorb water is suppressed even in a case where they are used for an absorbent article (in a case where the water-absorbent resin particles are pulverized in a manufacturing process of an absorbent article). A dissolved content after pulverization is preferably 1% by mass or more from the viewpoint of improving shape retainability of an absorbent when it is moisturized.

An amount of adhesion of the pulverized particles to a filter paper after the particles absorb an aqueous solution of 0.9% by mass NaCl at 25°C is preferably 6.0 g or less, 5.0 g or less, or 4.0 g or less. The amount of adhesion is measured by a method described in Examples to be described later.

A dissolved content of the water-absorbent resin particles (before pulverization) according to the present embodiment may be, for example, 40% by mass or less, 35% by mass or less, 30% by mass or less, 25% by mass or less, 20% by mass or less, or 18% by mass or less. It is desirable that a dissolved content before pulverization of the water-absorbent resin particles according to the present embodiment be as low as possible, but it may be, for example, 1% by mass or more, 5% by mass or more, 8% by mass or more, 10% by mass or more, 11% by mass or more, 12% by mass or more, or 13% by mass or more. In a case where a dissolved content before pulverization is within these ranges, a dissolved content after pulverization is easily set to 40% by mass or less. A dissolved content before pulverization is preferably 1% by mass or more from the viewpoint of improving shape retainability of an absorbent when it is moisturized.

A dissolved content before or after pulverization of the particles is measured by the following method. 500 g of an aqueous solution of 0.9% by mass NaCl is put into a 500 mL beaker and stirred at 600 rpm. 2 g of the particles is put into the beaker, stirred for 3 hours, and then filtered through a 75 $\mu$m standard sieve, and a filtrate is recovered. The obtained filtrate is further suction-filtered using a type 6 filter paper defined in JIS P 3801. 80 g of the filtrate obtained by suction filtration is weighed into a pre-weighed 100 mL beaker and dried with a hot air dryer at 140°C for 15 hours, and a mass (Wa) of a solid content of the filtrate is measured. A mass (Wb) of a solid content of a filtrate is measured by the same procedure without using the particles. A dissolved content is calculated by the following formula.

$$\text{Dissolved content (\% by mass)} = [((Wa - Wb)/80) \times 500/2] \times 100$$

Proportions of particles having a particle size of 300 $\mu$m or less before and after pulverization are measured using a sieve having an aperture of 300 $\mu$m. Median particle sizes before and after pulverization are measured by a sieving method. More specifically, the measurement is performed by a method described in Examples to be described later.

A water retention capacity of the water-absorbent resin particles according to the present embodiment for a physiological saline solution may be 20 g/g or more, 25 g/g or more, 27 g/g or more, 30 g/g or more, 32 g/g or more, 35 g/g or more, 37 g/g or more, 39 g/g or more, or 40 g/g or more, from the viewpoint of appropriately increasing an absorption capacity of an absorbent. A water retention capacity of the water-absorbent resin particles for a physiological saline solution may be 70 g/g or less, 65 g/g or less, 60 g/g or less, 57 g/g or less, 55 g/g or less, 52 g/g or less, 50 g/g or less, 47 g/g or less, 45 g/g or less, or 43 g/g or less. A water retention capacity for a physiological saline solution may be 20 to 70 g/g, 25 to 65 g/g, 27 to 60 g/g, 30 to 57 g/g, or 32 to 55 g/g. Furthermore, a water retention capacity for a physiological saline solution may be 30 to 70 g/g, 32 to 65 g/g, 35 to 65 g/g, 37 to 60 g/g, 39 to 60 g/g, 39 to 55 g/g, 40 to 55 g/g, or 40 to 50 g/g. The water retention capacity for a physiological saline solution is measured by the following method. A cotton bag (cotton broadcloth No. 60, 100 mm in width × 200 mm in length) into which 2 g of the water-absorbent resin particles has been weighed is placed in a beaker having a capacity of 500 mL. 500 g of an aqueous solution of 0.9% by mass sodium chloride (physiological saline solution) is poured into the cotton bag containing the water-absorbent resin particles at once so that a lump cannot be produced. The upper part of the cotton bag is bound with a rubber band and left to stand for 30 minutes, and thereby the water-absorbent resin particles are swollen. The cotton bag after an elapse of 30 minutes is dehydrated for 1 minute using a dehydrator which has been set at a centrifugal force of 167 G, and a mass Wc (g) of the dehydrated cotton bag containing the swollen gel is measured. By performing the same operation without addition of the water-absorbent resin particles, a mass Wd (g) of an empty cotton bag upon moisturizing is measured, and a water retention capacity for a physiological saline solution is calculated by the following formula.

$$\text{Water retention capacity for physiological saline solution (g/g)} = (Wc - Wd)/2$$

The water-absorbent resin particles according to the present embodiment can contain, for example, a crosslinked polymer obtained by polymerization of monomers including ethylenically unsaturated monomers. That is, the water-absorbent resin particles according to the present embodiment can have a structural unit derived from ethylenically unsaturated monomers.

Examples of methods for polymerizing the monomers include a reverse-phase suspension polymerization method, an aqueous solution polymerization method, a bulk polymerization method, and a precipitation polymerization method. Among them, the reverse-phase suspension polymerization method or the aqueous solution polymerization method is preferable from the viewpoints of facilitating securement of favorable water absorption characteristics of the obtained water-absorbent resin particles and control of a polymerization reaction. Hereinbelow, a method for polymerizing ethylenically unsaturated monomers will be described with the reverse-phase suspension polymerization method as an example.

An ethylenically unsaturated monomer is preferably water-soluble. Examples thereof include (meth)acrylic acid and a salt thereof, 2-(meth)acrylamide-2-methylpropanesulfonic acid and a salt thereof, (meth)acrylamide, N,N-dimethyl (meth)acrylamide, 2-hydroxyethyl (meth)acrylate, N-methylol (meth)acrylamide, polyethylene glycol mono(meth)acrylate, N,N-diethylaminoethyl (meth)acrylate, N,N-diethylaminopropyl (meth)acrylate, and diethylaminopropyl (meth)acrylamide. In a case where an ethylenically unsaturated monomer has an amino group, the amino group may be quaternarized. A functional group such as a carboxyl group and an amino group, which is contained in the monomer, can function as a crosslinkable functional group in a surface crosslinking process to be described later. These ethylenically unsaturated monomers may be used alone or in a combination of two or more kinds thereof.

Among them, from the viewpoint of high industrial availability, the ethylenically unsaturated monomer preferably includes at least one compound selected from the group consisting of acrylic acid and a salt thereof, methacrylic acid and a salt thereof, acrylamide, methacrylamide, and N,N-dimethyl acrylamide, and more preferably includes at least one compound selected from the group consisting of acrylic acid and a salt thereof, methacrylic acid and a salt thereof, and acrylamide. The ethylenically unsaturated monomer even more preferably includes at least one compound selected from the group consisting of acrylic acid and a salt thereof, and methacrylic acid and a salt thereof, from the viewpoint of further enhancing water absorption characteristics.

For the monomer, a monomer other than the above-mentioned ethylenically unsaturated monomers may be partially used. Such a monomer can be used by, for example, being mixed with an aqueous solution containing the ethylenically unsaturated monomers. It is preferable that a usage amount of the ethylenically unsaturated monomers be 70 to 100 mol% with respect to a total amount of the monomers. Among the examples, it is more preferable that an amount of (meth)acrylic acid and a salt thereof be 70 to 100 mol% with respect to the total amount of the monomers.

Usually, the ethylenically unsaturated monomers are suitably used in a form of an aqueous solution. In general, it is sufficient for a concentration of the ethylenically unsaturated monomers in an aqueous solution containing the ethylenically unsaturated monomers (hereinafter, referred to as an aqueous solution of monomers) to be 20% by mass or more and a saturated concentration or less, and it is preferably 25% to 70% by mass, and is more preferably 30% to 55% by mass. Examples of water to be used include tap water, distilled water, and ion exchange water.

In a case where ethylenically unsaturated monomers to be used have an acidic group, an aqueous solution of monomers may be used after neutralizing this acidic group with an alkaline neutralizing agent. From the viewpoint of increasing an osmotic pressure of the obtained water-absorbent resin particles and thereby further enhancing water absorption characteristics such as a water retention capacity, a degree of neutralization in the ethylenically unsaturated monomers by the alkaline neutralizing agent is 10 to 100 mol%, is preferably 50 to 90 mol%, and is more preferably 60 to 80 mol% of the acidic group in the ethylenically unsaturated monomers. Examples of alkaline neutralizing agents include alkali metal salts such as sodium hydroxide, sodium carbonate, sodium hydrogen carbonate, potassium hydroxide, and potassium carbonate; and ammonia. These alkaline neutralizing agents may be used in a form of an aqueous solution to simplify a neutralizing operation. The above-mentioned alkaline neutralizing agents may be used alone or in combination of two or more kinds thereof. Neutralization of the acidic groups in the ethylenically unsaturated monomers can be performed by, for example, adding an aqueous solution of sodium hydroxide, potassium hydroxide, or the like dropwise to the aqueous solution of monomers and mixing them.

In the reverse-phase suspension polymerization method, an aqueous solution of monomers is dispersed in a hydrocarbon dispersion medium in the presence of a surfactant, and polymerization of ethylenically unsaturated monomers is performed using a radical polymerization initiator or the like. As the radical polymerization initiator, it is possible to use, for example, a water-soluble radical polymerization initiator. An internal crosslinking agent may be used in the polymerization.

Examples of surfactants include nonionic surfactants and anionic surfactants. Examples of nonionic surfactants include sorbitan fatty acid esters, (poly)glycerin fatty acid esters (where "(poly)" means both of a case with the prefix "poly" and a case without the prefix "poly," and the same applies hereinbelow), sucrose fatty acid esters, polyoxyethylene sorbitan fatty acid esters, polyoxyethylene glycerin fatty acid esters, sorbitol fatty acid esters, polyoxyethylene sorbitol fatty acid esters, polyoxyethylene alkyl ethers, polyoxyethylene alkylphenyl ethers, polyoxyethylene castor oil, polyoxyethylene hydrogenated castor oil, alkylallyl formaldehyde condensed polyoxyethylene ethers, polyoxyethylene polyoxypropylene block copolymers, polyoxyethylene polyoxypropyl alkyl ethers, and polyethylene glycol fatty acid esters. Examples of anionic surfactants include fatty acid salts, alkylbenzene sulfonate, alkylmethyl taurate, polyoxyethylene alkylphenyl ether sulfuric acid ester salts, polyoxyethylene alkyl ether sulfonic acid salts, phosphoric acid esters of polyoxyethylene alkyl ethers, and phosphoric acid esters of polyoxyethylene alkyl allyl ethers. Among them, the surfactant preferably includes at least one compound selected from the group consisting of sorbitan fatty acid esters, polyglycerin fatty acid esters, and sucrose fatty acid esters, from the viewpoints that then, a state of a W/O type reverse-phase suspension becomes favorable, water-absorbent resin particles are likely to be obtained with suitable particle sizes, and industrial availability becomes high. Furthermore, the surfactant more preferably includes sucrose fatty acid esters from the viewpoint that water absorption characteristics of the obtained water-absorbent resin particles are then improved. These surfactants may be used alone or in combination of two or more kinds thereof.

An amount of the surfactant is preferably 0.05 to 10 parts by mass, is more preferably 0.08 to 5 parts by mass, and is even more preferably 0.1 to 3 parts by mass, with respect to 100 parts by mass of the aqueous solution of the ethylenically unsaturated monomers, from the viewpoint that a sufficient effect is obtained within these usage amounts, and these amounts are economic.

Furthermore, in the reverse-phase suspension polymerization, a polymeric dispersant may be used in combination with the above-mentioned surfactant.

Examples of polymeric dispersants include maleic anhydride-modified polyethylene, maleic anhydride-modified polypropylene, a maleic anhydride-modified ethylene-propylene copolymer, a maleic anhydride-modified EPDM (ethylene propylene diene terpolymer), maleic anhydride-modified polybutadiene, a maleic anhydride-ethylene copolymer, a maleic anhydride-propylene copolymer, a maleic anhydride-ethylene-propylene copolymer, a maleic anhydride-butadiene copolymer, polyethylene, polypropylene, an ethylene-propylene copolymer, oxidized polyethylene, oxidized polypropylene, an oxidized ethylene-propylene copolymer, an ethylene-acrylic acid copolymer, ethyl cellulose, ethyl hydroxyethyl cellulose, and the like. Among these polymeric dispersants, particularly from the viewpoint of dispersion stability of monomers, it is preferable to use maleic anhydride-modified polyethylene, maleic anhydride-modified polypropylene, a maleic anhydride-modified ethylene-propylene copolymer, a maleic anhydride-ethylene copolymer, a maleic anhydride-propylene copolymer, a maleic anhydride-ethylene-propylene copolymer, polyethylene, polypropylene, an ethylene-propylene copolymer, oxidized polyethylene, oxidized polypropylene, and an oxidized ethylene-propylene copolymer. These polymeric dispersants may be used alone or in combination of two or more kinds thereof.

An amount of the polymeric dispersant is preferably 0.05 to 10 parts by mass, is more preferably 0.08 to 5 parts by mass, and is even more preferably 0.1 to 3 parts by mass, with respect to 100 parts by mass of the aqueous solution of the ethylenically unsaturated monomers, from the viewpoint that a sufficient effect is obtained within these usage amounts, and these amounts are economic.

A radical polymerization initiator is preferably water-soluble. Examples thereof include persulfates such as potassium persulfate, ammonium persulfate, and sodium persulfate; peroxides such as methyl ethyl ketone peroxide, methyl isobutyl ketone peroxide, di-t-butyl peroxide, t-butyl cumyl peroxide, t-butyl peroxyacetate, t-butyl peroxyisobutyrate, t-butyl peroxypivalate, and hydrogen peroxide; and azo compounds such as 2,2'-azobis(2-amidinopropane) dihydrochloride, 2,2'-azobis[2-(N-phenylamidino)propane] dihydrochloride, 2,2'-azobis[2-(N-allylamidino)propane] dihydrochloride, 2,2'-azobis[2-(2-imidazolin-2-yl)propane] dihydrochloride, 2,2'-azobis{2-[1-(2-hydroxyethyl)-2-imidazolin-2-yl]propane} dihydrochloride, 2,2'-azobis{2-methyl-N-[1,1-bis(hydroxymethyl)-2-hydroxyethyl]propi onamide}, 2,2'-azobis[2-methyl-N-(2-hydroxyethyl)-propionamide], and 4,4'-azobis(4-cyanovaleric acid). Among them, potassium persulfate, ammonium persulfate, sodium persulfate, 2,2' -azobis(2-amidinopropane) dihydrochloride, 2,2'-azobis[2-(2-imidazolin-2-yl)propane] dihydrochloride, and 2,2'-azobis{2-[1-(2-hydroxyethyl)-2-imidazolin-2-yl]propane} dihydrochloride are preferred. These radical polymerization initiators may be used alone or in combination of two or more kinds thereof.

A usage amount of the radical polymerization initiator may be 0.00005 to 0.01 moles with respect to 1 mole of the ethylenically unsaturated monomers. A case in which a usage amount of the radical polymerization initiator is 0.00005 moles or more is efficient, because then a polymerization reaction is not required to be performed for a long period of time. In a case where a usage amount thereof is 0.01 moles or less, a rapid polymerization reaction is unlikely to occur.

The radical polymerization initiator can also be used as a redox polymerization initiator when it is used in combination with a reducing agent such as sodium sulfite, sodium hydrogen sulfite, ferrous sulfate, and L-ascorbic acid.

In a polymerization reaction, a chain transfer agent may be contained in an aqueous solution of the ethylenically unsaturated monomers used for the polymerization. Examples of chain transfer agents include hypophosphites, thiols, thiolic acids, secondary alcohols, and amines.

Furthermore, a thickener may be contained in the aqueous solution of the ethylenically unsaturated monomers used for the polymerization to control a particle size of the water-absorbent resin particles.

As the thickener, it is possible to use, for example, hydroxyethyl cellulose, hydroxypropyl cellulose, methyl cellulose, carboxymethyl cellulose, polyethylene glycol, polyacrylamide, polyethyleneimine, dextrin, sodium alginate, polyvinyl alcohol, polyvinylpyrrolidone, polyethylene oxide, and the like. In a case where stirring speeds in the polymerization are the same, a median particle size of particles to be obtained is likely to become large as a viscosity of the aqueous solution of the ethylenically unsaturated monomers becomes high.

The hydrocarbon dispersion medium may include at least one compound selected from the group consisting of a chained aliphatic hydrocarbon having 6 to 8 carbon atoms and an alicyclic hydrocarbon having 6 to 8 carbon atoms. Examples of hydrocarbon dispersion media include chained aliphatic hydrocarbons such as n-hexane, n-heptane, 2-methylhexane, 3-methylhexane, 2,3-dimethylpentane, 3-ethylpentane, and n-octane; alicyclic hydrocarbons such as cyclohexane, methylcyclohexane, cyclopentane, methylcyclopentane, trans-1,2-dimethylcyclopentane, cis-1,3-dimethylcyclopentane, and trans-1,3-dimethylcyclopentane; and aromatic hydrocarbons such as benzene, toluene, and xylene. These hydrocarbon dispersion media may be used alone or in combination of two or more kinds thereof. For the hydrocarbon dispersion medium, n-heptane, cyclohexane, or both n-heptane and cyclohexane may be contained, from the viewpoints of high industrial availability and stable qualities. Furthermore, from the same viewpoints, as a mixture of the hydrocarbon dispersion media, for example, a commercially available Exxsol Heptane (manufactured by ExxonMobil Chemical: containing n-heptane and 75% to 85% of hydrocarbons of isomers thereof) may be used.

A usage amount of the hydrocarbon dispersion medium is preferably 30 to 1,000 parts by mass, is more preferably 40 to 500 parts by mass, and is even more preferably 50 to 300 parts by mass, with respect to 100 parts by mass of the aqueous solution of monomers, from the viewpoint that polymerization heat is then appropriately removed, and thereby a polymerization temperature is easily controlled. In a case where a usage amount of the hydrocarbon dispersion medium is 30 parts by mass or more, there is a tendency that it becomes easy to control a polymerization temperature. In a case where a usage amount of the hydrocarbon dispersion medium is 1,000 parts by mass or less, there is a tendency that productivity of polymerization is improved, which is economic.

In general, internal crosslinking may occur by self-crosslinking upon the polymerization, but internal crosslinking may be carried out by further using an internal crosslinking agent, and thereby water absorption characteristics of the water-absorbent resin particles may be controlled. Examples of internal crosslinking agents to be used include di- or tri(meth)acrylic acid esters of polyols such as ethylene glycol, propylene glycol, trimethylolpropane, glycerin, polyoxyethylene glycol, polyoxypropylene glycol, and polyglycerin; unsaturated polyesters obtained by reacting the above mentioned polyols with unsaturated acids such as maleic acid and fumaric acid; bis(meth)acrylamides such as N,N'-methylenebis(meth)acrylamide; di- or tri(meth)acrylic acid esters obtained by reacting a polyepoxide with (meth)acrylic acid; carbamyl di(meth)acrylate esters obtained by reacting a polyisocyanate such as tolylene diisocyanate and hexamethylene diisocyanate with hydroxyethyl (meth)acrylate; compounds having two or more polymerizable unsaturated groups, such as allylated starch, allylated cellulose, diallyl phthalate, N, N', N"-triallyl isocyanurate, and divinylbenzene; polyglycidyl compounds such as (poly)ethylene glycol diglycidyl ether, (poly)propylene glycol diglycidyl ether, (poly)glycerin diglycidyl ether, (poly)glycerin triglycidyl ether, (poly)propylene glycol polyglycidyl ether, and polyglycerol polyglycidyl ether; haloepoxy compounds such as epichlorohydrin, epibromohydrin, and $\alpha$-methyl epichlorohydrin; and compounds having two or more reactive functional groups, such as isocyanate compounds including, for example, 2,4-tolylene diisocyanate and hexamethylene diisocyanate. Among these internal crosslinking agents, it is preferable to use a polyglycidyl compound, it is more preferable to use a diglycidyl ether compound, and it is particularly preferable to use (poly)ethylene glycol diglycidyl ether, (poly)propylene glycol diglycidyl ether, and (poly)glycerin diglycidyl ether. These crosslinking agents may be used alone or in combination of two or more kinds thereof.

An amount of the internal crosslinking agent is preferably 0 to 0.03 moles, is more preferably 0.00001 to 0.01 moles, and is even more preferably 0.00002 to 0.005 moles, per 1 mole of the ethylenically unsaturated monomer, from the viewpoints of inhibiting water-soluble properties by appropriately crosslinking the obtained polymer, and exhibiting a sufficient water absorption capacity. Furthermore, in a case where an amount of the internal crosslinking agent is within the above range, it is easy to obtain water-absorbent resin particles having a dissolved content after pulverization of 40% by mass or less.

The reverse-phase suspension polymerization can be performed in a water-in-oil system by mixing ethylenically unsaturated monomers, a radical polymerization initiator, a surfactant, a polymeric dispersant, a hydrocarbon dispersion medium, and the like (and an internal crosslinking agent as necessary), and heating the mixture under stirring.

When performing the reverse-phase suspension polymerization, an aqueous solution of monomers which contains ethylenically unsaturated monomers is dispersed in a hydrocarbon dispersion medium in the presence of a surfactant and if necessary, a polymeric dispersant. In this case, a timing of adding the surfactant or the polymeric dispersant before the start of the polymerization reaction may be either before or after the addition of the aqueous solution of monomers.

Among them, it is preferable to carry out the polymerization after dispersing the aqueous solution of monomers in the hydrocarbon dispersion medium in which the polymeric dispersant has been dispersed, and then further dispersing the surfactant in the hydrocarbon dispersion medium, from the viewpoint that an amount of the hydrocarbon dispersion medium remaining in the obtained water-absorbing resin can then be easily reduced.

Such reverse-phase suspension polymerization can be carried out in one stage or in multiple stages of two or more stages. It is preferably carried out in two or three stages from the viewpoint of increasing productivity. Furthermore, by performing the reverse-phase suspension polymerization in multiple stages, preferably in two stages, it becomes easy to obtain water-absorbent resin particles having a particle size suitable for an absorbent article.

In a case where reverse-phase suspension polymerization is carried out in multiple stages of two or more stages, it is sufficient for stages after a second stage of reverse-phase suspension polymerization to be carried out in the same manner as in a first stage of reverse-phase suspension polymerization by adding ethylenically unsaturated monomers to a reaction mixture obtained in the first stage of polymerization reaction and mixing them, after performing the first stage of reverse-phase suspension polymerization. In reverse-phase suspension polymerization in each stage after the second stage, it is preferable to carry out reverse-phase suspension polymerization by adding, in addition to ethylenically unsaturated monomers, the above-mentioned radical polymerization initiator and internal crosslinking agent within a range of molar ratios of the respective components to the ethylenically unsaturated monomers, based on an amount of ethylenically unsaturated monomers added during reverse-phase suspension polymerization in each stage after the second stage. In the reverse-phase suspension polymerization in each stage after the second stage, a case in which an amount of the internal crosslinking agent is small makes it easy to obtain water-absorbent resin particles having a dissolved content after pulverization of 40% by mass or less.

A temperature for the polymerization reaction varies depending on radical polymerization initiators used, and it is preferably 20°C to 150°C, and is more preferably 40°C to 120°C, from the viewpoint that the polymerization is then promptly performed, which shortens a polymerization time, and thereby economic efficiency increases, and that polymerization heat is then easily removed, and thereby the reaction is smoothly performed. A reaction time is generally 0.5 to 4 hours. Completion of the polymerization reaction can be confirmed from, for example, stop of temperature rising in the reaction system. Accordingly, a polymer of ethylenically unsaturated monomers is generally obtained in a state of a hydrous gel.

After the polymerization, post-polymerization crosslinking may be carried out by adding a crosslinking agent to the obtained hydrous gel polymer and heating them. In a case where the post-polymerization crosslinking is performed, it is possible to easily obtain water-absorbent resin particles exhibiting suitable water absorption characteristics. Furthermore, a dissolved content after pulverization is easily set to 40% by mass or less.

Examples of crosslinking agents for performing the post-polymerization crosslinking include polyols such as ethylene glycol, propylene glycol, 1,4-butanediol, trimethylolpropane, glycerin, polyoxyethylene glycol, polyoxypropylene glycol, and polyglycerin; compounds having two or more epoxy groups, such as (poly)ethylene glycol diglycidyl ether, (poly)propylene glycol diglycidyl ether, and (poly)glycerin diglycidyl ether; haloepoxy compounds such as epichlorohydrin, epibromohydrin, and $\alpha$-methyl epichlorohydrin; compounds having two or more isocyanate groups such as 2,4-tolylene diisocyanate and hexamethylene diisocyanate; oxazoline compounds such as 1,2-ethylenebisoxazoline; carbonate compounds such as ethylene carbonate; and hydroxyalkylamide compounds such as bis[N,N-di($\beta$-hydroxyethyl)]adipamide. Among them, polyglycidyl compounds such as (poly)ethylene glycol diglycidyl ether, (poly)glycerin diglycidyl ether, (poly)glycerin triglycidyl ether, (poly)propylene glycol polyglycidyl ether, and polyglycerol polyglycidyl ether are preferable. These crosslinking agents may be used alone or in combination of two or more kinds thereof.

An amount of the crosslinking agent used for the post-polymerization crosslinking is preferably 0 to 0.03 moles, is more preferably 0 to 0.01 moles, and is even more preferably 0.00001 to 0.005 moles, per 1 mole of the ethylenically unsaturated monomer, from the viewpoint of exhibiting suitable water absorption characteristics by appropriately crosslinking the obtained hydrous gel polymer. In a case where an amount of the crosslinking agent used for the post-polymerization crosslinking is within the above range, a dissolved content after pulverization of the obtained water-absorbent resin particles is easily set to 40% by mass or less.

It is sufficient for a timing for adding the post-polymerization crosslinking to be after polymerization of ethylenically unsaturated monomers used for the polymerization. In a case of multi-stage polymerization, the crosslinking agent is preferably added after the multi-stage polymerization. From the viewpoint of a water content (to be described later), it is preferable to add the crosslinking agent for the post-polymerization crosslinking within a region of [water content immediately after polymerization $\pm$ 3% by mass], in consideration of heat generation during and after polymerization, retention due to process delay, system opening when a crosslinking agent is added, and fluctuation in water content due to addition of water associated with addition of a crosslinking agent.

Subsequently, drying is performed to remove water from the obtained hydrous gel polymer. By drying, polymer particles containing the polymer of ethylenically unsaturated monomers are obtained. Examples of drying methods include a method (a) in which the hydrous gel polymer in a state of being dispersed in a hydrocarbon dispersion medium is subjected to azeotropic distillation by heating from the outside, and the hydrocarbon dispersion medium is refluxed to remove water; a method (b) in which the hydrous gel polymer is taken out by decantation and dried under reduced pressure; and a method (c) in which the hydrous gel polymer is separated by filtration with a filter and dried under reduced pressure. Among them, the method (a) is preferably used for its simplicity in a production process.

Control over a particle size of the water-absorbent resin particle can be performed, for example, by adjusting a rotational speed of a stirrer during the polymerization reaction or by adding a powdery inorganic flocculating agent to the system after the polymerization reaction or at an initial time of drying. A particle size of the obtained water-absorbent resin particle can be increased by adding the flocculating agent. Examples of powdery inorganic flocculating agents include silica, zeolite, bentonite, aluminum oxide, talc, titanium dioxide, kaolin, clay, and hydrotalcite. Among them, silica, aluminum oxide, talc, or kaolin is preferable from the viewpoint of a flocculation effect.

In the reverse-phase suspension polymerization, examples of methods of adding the powdery inorganic flocculating agent include a method in which a powdery inorganic flocculating agent is dispersed in a hydrocarbon dispersion medium of the same kind as that used in the polymerization, or water in advance, and then the mixture is mixed into a hydrocarbon dispersion medium containing a hydrous gel polymer under stirring.

An amount of the powdery inorganic flocculating agent added is preferably 0.001 to 1 part by mass, is more preferably 0.005 to 0.5 parts by mass, and is even more preferably 0.01 to 0.2 parts by mass, with respect to 100 parts by mass of ethylenically unsaturated monomers provided for the polymerization. By setting an amount of the powdery inorganic flocculating agent added to be within the above range, it is easy to obtain water-absorbent resin particles having a desired particle size distribution.

In the production of the water-absorbent resin particles according to the present embodiment, a surface portion of the hydrous gel polymer is preferably crosslinked (surface-crosslinked) using a crosslinking agent in the drying process or any of subsequent processes. By performing surface crosslinking, it is easy to control water absorption characteristics of the water-absorbent resin particles. Furthermore, a dissolved content after pulverization is easily set to 40% by mass or less. The surface crosslinking is preferably performed at a timing when the hydrous gel polymer has a specific water content. A timing of the surface crosslinking is preferably a time point at which a water content of the hydrous gel polymer is 5% to 50% by mass, is more preferably a time point at which a water content thereof is 10% to 40% by mass, and is even more preferably a time point at which a water content thereof is 15% to 35% by mass.

A water content (% by mass) of the hydrous gel polymer is calculated by the following formula.

$$\text{Water content} = [\text{Ww}/(\text{Ww} + \text{Ws})] \times 100$$

Ww: An amount of water of a hydrous gel polymer obtained by adding an amount of water used, as desired, upon mixing a powdery inorganic flocculating agent, a surface crosslinking agent, and the like to an amount obtained by subtracting an amount of water extracted to the outside of the system by the drying process from an amount of water contained in an aqueous liquid before polymerization in the all polymerization processes.

Ws: A solid fraction calculated from an amount of materials introduced, such as ethylenically unsaturated monomers, a crosslinking agent, and an initiator, each of which constitutes the hydrous gel polymer.

Examples of surface crosslinking agents for performing surface crosslinking include compounds having two or more reactive functional groups. Examples thereof include polyols such as ethylene glycol, propylene glycol, 1,4-butanediol, trimethylolpropane, glycerin, polyoxyethylene glycol, polyoxypropylene glycol, and polyglycerin; polyglycidyl compounds such as (poly)ethylene glycol diglycidyl ether, (poly)glycerin diglycidyl ether, (poly)glycerin triglycidyl ether, trimethylolpropane triglycidyl ether, (poly)propylene glycol polyglycidyl ether, and (poly)glycerol polyglycidyl ether; haloepoxy compounds such as epichlorohydrin, epibromohydrin, and $\alpha$-methyl epichlorohydrin; isocyanate compounds such as 2,4-tolylene diisocyanate and hexamethylene diisocyanate; oxetane compounds such as 3-methyl-3-oxetane methanol, 3-ethyl-3-oxetane methanol, 3-butyl-3-oxetane methanol, 3-methyl-3-oxetane ethanol, 3-ethyl-3-oxetane ethanol, and 3-butyl-3-oxetane ethanol; oxazoline compounds such as 1,2-ethylenebisoxazoline; carbonate compounds such as ethylene carbonate; and hydroxyalkylamide compounds such as bis[N,N-di($\beta$-hydroxyethyl)]adipamide. Among them, polyglycidyl compounds such as (poly)ethylene glycol diglycidyl ether, (poly)glycerin diglycidyl ether, (poly)glycerin triglycidyl ether, (poly)propylene glycol polyglycidyl ether, and polyglycerol polyglycidyl ether are more preferable. These surface crosslinking agents may be used alone or in combination of two or more kinds thereof.

In general, an amount of the surface crosslinking agent is preferably 0.00001 to 0.02 moles, is more preferably 0.00005 to 0.01 moles, and is even more preferably 0.0001 to 0.005 moles in a molar ratio, with respect to 1 mole of the ethylenically unsaturated monomer used in the polymerization, from the viewpoint of exhibiting suitable water absorption characteristics by appropriately crosslinking the obtained hydrous gel polymer.

A usage amount of the surface crosslinking agent is preferably 0.00001 moles or more from the viewpoint of sufficiently increasing a crosslinking density in a surface portion of the water-absorbent resin particles and thereby enhancing gel strength of the water-absorbent resin particles. A usage amount thereof is preferably 0.02 moles or less from the viewpoint of increasing a water retention capacity of the water-absorbent resin particles. Furthermore, in a case where a usage amount of the surface crosslinking agent is within the above range, a dissolved content after pulverization of the obtained water-absorbent resin particles is easily set to 40% by mass or less.

It is possible to obtain polymer particles, which are a surface-crosslinked dried product, by distilling off water and the hydrocarbon dispersion medium by a known method after the surface crosslinking reaction.

The water-absorbent resin particles according to the present embodiment may be composed of only the polymer particles, but they can further contain, for example, various additional components selected from gel stabilizers, metal chelating agents (ethylenediaminetetraacetic acid and its salts, diethylenetriaminepentaacetic acid and its salts, for example, diethylenetriaminepentaacetic acid pentasodium, and the like), flowablility improvers (lubricants), and the like. The additional components may be disposed inside the polymer particles, on a surface of the polymer particles, or both of the inside and on the surface thereof. As the additional component, flowablility improvers (lubricants) are preferable, and among them, inorganic particles are more preferable. Examples of inorganic particles include silica particles such as amorphous silica.

The water-absorbent resin particles may contain a plurality of inorganic particles disposed on the surface of the polymer particles. The inorganic particles can be disposed on the surface of the polymer particles by, for example, mixing the polymer particles and the inorganic particles. These inorganic particles may be silica particles such as amorphous silica. In a case where the water-absorbent resin particles contain inorganic particles disposed on the surface of the polymer particles, a ratio of the inorganic particles to a mass of the polymer particles may be 0.2% by mass or more, 0.5% by mass or more, 1.0% by mass or more, or 1.5% by mass or more, and it may be 5.0% by mass or less or 3.5% by mass or less. The inorganic particles referred to herein generally have a minute size as compared with a size of the polymer particles. For example, an average particle size of the inorganic particles may be 0.1 to 50 $\mu$m, 0.5 to 30 $\mu$m, or 1 to 20 $\mu$m. The average particle size referred to herein can be a value measured by a dynamic light scattering method or a laser diffraction/scattering method. In a case where an amount of the inorganic particles added is within the above range, it is easy to obtain water-absorbent resin particles having favorable water absorption characteristics and a suitable numerical value for a dissolved content after pulverization.

The water-absorbent resin particles according to the present embodiment have better absorbency for body fluids such as urine and blood, and they can be applied to, for example, the fields of sanitary products such as paper diapers, sanitary napkins, and tampons, and animal excrement treatment materials such as pet sheets, and dog or cat litters.

The water-absorbent resin particles according to the present embodiment can be suitably used for an absorbent. The absorbent according to the present embodiment includes the above-mentioned water-absorbent resin particles. The absorbent may further include, for example, a fibrous material.

A mass proportion of the water-absorbent resin particles in the absorbent may be 2% by mass to 100% by mass, is preferably 10% by mass to 80% by mass, and is more preferably 20% by mass to 60% by mass, with respect to a total of the water-absorbent resin particles and the fibrous material. The configuration of the absorbent may be, for example, a form in which water-absorbent resin particles and the fibrous materials are uniformly mixed, a form in which water-absorbent resin particles are held between fibrous materials formed in a sheet shape or a layer shape, or another form.

A content of the water-absorbent resin particles in the absorbent is preferably 100 to 1,000 g, is more preferably 150 to 800 g, and is even more preferably 200 to 700 g, per 1 m$^2$ of the absorbent from the viewpoint of easily obtaining a sufficient water absorption performance. A content of the fibrous material in the absorbent is preferably 50 to 800 g, is more preferably 100 to 600 g, and is even more preferably 150 to 500 g, per 1 m$^2$ of the absorbent from the viewpoint of easily obtaining a sufficient water absorption performance.

Examples of fibrous materials include finely pulverized wood pulp; cotton; cotton linter; rayon; cellulose-based fibers such as cellulose acetate; and synthetic fibers such as polyamides, polyesters, and polyolefins. The fibrous material may be a mixture of the above-mentioned fibers.

Fibers may be adhered to each other by adding an adhesive binder to the fibrous material in order to enhance shape retention properties before or during use of the absorbent. Examples of adhesive binders include thermal bonding synthetic fibers, hot-melt adhesives, and adhesive emulsions.

Examples of thermal bonding synthetic fibers include full-melt binders such as polyethylene, polypropylene, and an ethylene-propylene copolymer; and partial-melt binders formed of polypropylene and polyethylene in a side-by-side or core-and-sheath configuration. In the above-mentioned partial-melt binders, only a polyethylene portion is thermal-bonded. Examples of hot-melt adhesives include a blend of a base polymer such as an ethylene-vinyl acetate copolymer, a styrene-isoprene-styrene block copolymer, a styrene-butadiene-styrene block copolymer, a styrene-ethylene-butylene-styrene block copolymer, a styrene-ethylene-propylene-styrene block copolymer, and an amorphous polypropylene with a viscosity imparting agent, a plasticizer, an antioxidant, or the like.

Examples of adhesive emulsions include polymers of at least one or more monomers selected from the group consisting of methyl methacrylate, styrene, acrylonitrile, 2-ethylhexyl acrylate, butyl acrylate, butadiene, ethylene, and vinyl acetate. These adhesive binders may be used alone or in combination of two or more kinds thereof.

The absorbent according to the present embodiment may further contain an inorganic powder (for example, amorphous silica), a deodorant, an antibacterial agent, a fragrance, and the like. In a case where the water-absorbent resin particles contain inorganic particles, the absorbent may contain an inorganic powder in addition to the inorganic particles in the water-absorbent resin particles.

A shape of the absorbent according to the present embodiment is not particularly limited, but it may be, for example, a sheet shape. A thickness of the absorbent (for example, a thickness of a sheet-shaped absorbent) may be, for example, 0.1 to 20 mm or 0.3 to 15 mm.

An absorbent article according to the present embodiment includes the absorbent according to the present embodiment. Examples of the absorbent article according to the present embodiment include a core wrap that retains the shape of the absorbent; a liquid-permeable sheet disposed on the outermost part on a side from which an absorption target liquid is infiltrated; a liquid-impermeable sheet disposed on the outermost part on a side opposite to the side from which the absorption target liquid is infiltrated; and the like. Examples of the absorbent article include diapers (for example, paper diapers), toilet training pants, incontinence pads, sanitary products (sanitary napkins, tampons, and the like), sweat pads, pet sheets, portable toilet members, animal excrement treatment materials, and the like. Since the absorbent article according to the present embodiment contains the water-absorbent resin particles, even in a case where some of the water-absorbent resin particles are pulverized, stickiness after they absorb water is small, and discomfort when use is reduced.

Fig. 1 is a cross-sectional view showing an example of an absorbent article. An absorbent article 100 shown in Fig. 1 includes an absorbent 10, core wraps 20a and 20b, a liquid-permeable sheet 30, and a liquid-impermeable sheet 40. In the absorbent article 100, the liquid-impermeable sheet 40, the core wrap 20b, the absorbent 10, the core wrap 20a, and the liquid-permeable sheet 30 are laminated in this order. In Fig. 1, there is a portion shown to be a gap between the members, but the members may be in close contact with each other without the gap.

The absorbent 10 has water-absorbent resin particles 10a according to the present embodiment and a fiber layer 10b containing a fibrous material. The water-absorbent resin particles 10a are dispersed in the fiber layer 10b.

The core wrap 20a is disposed on one surface side of the absorbent 10 (an upper side of the absorbent 10 in Fig. 1) in a state of being in contact with the absorbent 10. The core wrap 20b is disposed on the other surface side of the absorbent 10 (a lower side of the absorbent 10 in Fig. 1) in a state of being in contact with the absorbent 10. The absorbent 10 is disposed between the core wrap 20a and the core wrap 20b. Examples of the core wraps 20a and 20b include tissues, non-woven fabrics, and the like. The core wrap 20a and the core wrap 20b each have, for example, a main surface having the same size as that of the absorbent 10.

The liquid-permeable sheet 30 is disposed on the outermost part on a side from which an absorption target liquid is infiltrated. The liquid-permeable sheet 30 is disposed on the core wrap 20a in a state of being in contact with the core wrap 20a. Examples of the liquid-permeable sheet 30 include non-woven fabrics made of synthetic resin such as polyethylene, polypropylene, polyester, and polyamide, and porous sheets. The liquid-impermeable sheet 40 is disposed on the outermost part on a side opposite to the liquid-permeable sheet 30, in the absorbent article 100. The liquid-impermeable sheet 40 is disposed below the core wrap 20b in a state of being in contact with the core wrap 20b. Examples of the liquid-impermeable sheet 40 include sheets made of synthetic resins such as polyethylene, polypropylene, and polyvinyl chloride, and sheets made of a composite material of these synthetic resins and a non-woven fabric. The liquid-permeable sheet 30 and the liquid-impermeable sheet 40 each have, for example, a main surface wider than the main surface of the absorbent 10, and outer edges of the liquid-permeable sheet 30 and the liquid-impermeable sheet 40 respectively extend around the absorbent 10 and the core wraps 20a and 20b.

A magnitude relationship between the absorbent 10, the core wraps 20a and 20b, the liquid-permeable sheet 30, and the liquid-impermeable sheet 40 is not particularly limited, and it is appropriately adjusted according to usage applications and the like of the absorbent article. Furthermore, a method of retaining the shape of the absorbent 10 using the core wraps 20a and 20b is not particularly limited. The absorbent may be wrapped with a plurality of the core wraps as shown in Fig. 1, or the absorbent may be wrapped with one core wrap.

[Examples]

Hereinafter, the present invention will be described in more detail with reference to examples, but the present invention is not limited to these examples.

[Production of water-absorbent resin particles]

(Example 1) A cylindrical round-bottomed separable flask was prepared, which had an inner diameter of 11 cm and a capacity of 2 L, and was equipped with a reflux condenser, a dropping funnel, a nitrogen gas introduction tube, and as a stirrer, a stirring blade having four inclined paddle blades, each having a blade diameter of 5 cm, in a two-tier manner. 293 g of n-heptane as a hydrocarbon dispersion medium was weighed into this flask, and 0.736 g of a maleic anhydride-modified ethylene-propylene copolymer (HI-WAX 1105A, manufactured by Mitsui Chemicals, Inc.) as a polymeric dispersant were added thereinto. The reaction solution was heated to 80°C while being stirred to dissolve the dispersant, and then was cooled to 50°C.

Meanwhile, 92.0 g (1.03 moles) of an aqueous solution of 80.5% by mass acrylic acid as an ethylenically unsaturated monomer was weighed into a beaker with an internal capacity of 300 mL, and 147.7 g of an aqueous solution of 20.9% by mass sodium hydroxide was added dropwise thereto while cooling the beaker from the outside to perform neutralization to 75 mol%. Thereafter, 0.092 g of hydroxylethyl cellulose (HEC AW-15F, manufactured by Sumitomo Seika Chemicals Co., Ltd.) as a thickener, 0.0736 g (0.272 mmol) of potassium persulfate as a radical polymerization initiator, and 0.010 g (0.057 mmol) of ethylene glycol diglycidyl ether as an internal crosslinking agent were added and dissolved to prepare a first-stage aqueous liquid.

The prepared aqueous liquid was added into the separable flask and stirred for 10 minutes. Thereafter, a surfactant solution, which was obtained by dissolving 0.736 g of sucrose stearic acid ester with HLB 3 (RYOTO Sugar Ester S-370, manufactured by Mitsubishi-Chemical Foods Corporation) as a surfactant in 6.62 g of n-heptane through heating, was further added into the flask. While stirring the reaction solution at 550 rpm as a rotational speed of the stirrer, the inside of the system was sufficiently replaced with nitrogen. Thereafter, the flask was immersed in a water bath at 70°C to raise its temperature, and polymerization was performed for 60 minutes. Thereby, a first-stage polymerization slurry liquid was obtained.

Meanwhile, 128.8 g (1.43 moles) of an aqueous solution of 80.5% by mass acrylic acid as an ethylenically unsaturated monomer was weighed into another beaker with an internal capacity of 500 mL, and 159.0 g of an aqueous solution of 27% by mass sodium hydroxide was added dropwise thereto while cooling the beaker from the outside to perform neutralization to 75 mol%. Thereafter, 0.090 g (0.334 mmol) of potassium persulfate as a radical polymerization initiator was added and dissolved to prepare a second-stage aqueous liquid.

While stirring the aqueous liquid at 1,000 rpm as a rotational speed of the stirrer, the inside of the separable flask system was cooled to 25°C, and then a total amount of the second-stage aqueous liquid was added to the first-stage polymerization slurry liquid. The inside of the system was replaced with nitrogen for 30 minutes. Thereafter, the flask was immersed in a water bath again at 70°C to raise its temperature, and a polymerization reaction was performed for 60 minutes. After the polymerization, 0.580 g (0.067 mmol) of 2% by mass of ethylene glycol diglycidyl ether was added as a crosslinking agent to obtain a hydrous gel polymer.

Under stirring, 0.265 g of an aqueous solution of 45% by mass diethylenetriaminepentaacetic acid pentasodium was added to the hydrous gel polymer obtained after the second-stage polymerization. Thereafter, the flask was immersed in an oil bath set to 125°C, and 256.1 g of water was extracted out of the system while n-heptane was refluxed by azeotropic distillation with n-heptane and water. Thereafter, 4.42 g (0.507 mmol) of an aqueous solution of 2% by mass ethylene glycol diglycidyl ether as a surface crosslinking agent was added into the flask, and maintained at 83°C for 2 hours.

Thereafter, n-heptane was evaporated at 125°C, and the residue was dried to obtain a dried product (polymer particles). This dried product was passed through a sieve having an aperture of 850 $\mu$m, and 0.2% by mass of amorphous silica (Oriental Silicas Corporation, Tokusil NP-S) was mixed therewith. Thereby, 230.8 g of water-absorbent resin particles was obtained. A water retention capacity of the obtained water-absorbent resin particles for a physiological saline solution was 41 g/g.

(Example 2) A cylindrical round-bottomed separable flask was prepared, which had an inner diameter of 11 cm and a capacity of 2 L, and was equipped with a reflux condenser, a dropping funnel, a nitrogen gas introduction tube, and as a stirrer, a stirring blade having four inclined paddle blades, each having a blade diameter of 5 cm, in a two-tier manner. 293 g of n-heptane as a hydrocarbon dispersion medium was weighed into this flask, and 0.736 g of a maleic anhydride-modified ethylene-propylene copolymer (HI-WAX 1105A, manufactured by Mitsui Chemicals, Inc.) as a polymeric dispersant were added thereinto. The reaction solution was heated to 80°C while being stirred to dissolve the dispersant, and then was cooled to 50°C.

Meanwhile, 92.0 g (1.03 moles) of an aqueous solution of 80.5% by mass acrylic acid as an ethylenically unsaturated monomer was weighed into a beaker with an internal capacity of 300 mL, and 147.7 g of an aqueous solution of 20.9% by mass sodium hydroxide was added dropwise thereto while cooling the beaker from the outside to perform neutralization to 75 mol%. Thereafter, 0.092 g of hydroxylethyl cellulose (HEC AW-15F, manufactured by Sumitomo Seika Chemicals Co., Ltd.) as a thickener, 0.092 g (0.339 mmol) of 2,2'-azobis(2-amidinopropane) dihydrochloride and 0.018 g (0.068 mmol) of potassium persulfate as a radical polymerization initiator, and 0.037 g (0.211 mmol) of ethylene glycol diglycidyl ether as an internal crosslinking agent were added and dissolved to prepare a first-stage aqueous liquid.

The prepared aqueous liquid was added into the separable flask and stirred for 10 minutes. Thereafter, a surfactant solution, which was obtained by dissolving 0.736 g of sucrose stearic acid ester with HLB 3 (RYOTO Sugar Ester S-370, manufactured by Mitsubishi-Chemical Foods Corporation) as a surfactant in 6.62 g of n-heptane through heating, was further added into the flask. While stirring the reaction solution at 550 rpm as a rotational speed of the stirrer, the inside of the system was sufficiently replaced with nitrogen. Thereafter, the flask was immersed in a water bath at 70°C to raise its temperature, and polymerization was performed for 60 minutes. Thereby, a first-stage polymerization slurry liquid was obtained.

Meanwhile, 128.8 g (1.43 moles) of an aqueous solution of 80.5% by mass acrylic acid as an ethylenically unsaturated monomer was weighed into another beaker with an internal capacity of 500 mL, and 159.0 g of an aqueous solution of 27% by mass sodium hydroxide was added dropwise thereto while cooling the beaker from the outside to perform neutralization to 75 mol%. Thereafter, 0.129 g (0.475 mmol) of 2,2'-azobis(2-amidinopropane) dihydrochloride and 0.026 g (0.095 mmol) of potassium persulfate as a radical polymerization initiator were added and dissolved to prepare a second-stage aqueous liquid.

While stirring the aqueous liquid at 1,000 rpm as a rotational speed of the stirrer, the inside of the separable flask system was cooled to 25°C, and then a total amount of the second-stage aqueous liquid was added to the first-stage polymerization slurry liquid. The inside of the system was replaced with nitrogen for 30 minutes. Thereafter, the flask was immersed in a water bath again at 70°C to raise its temperature, and a polymerization reaction was performed for 60 minutes. After the polymerization, 0.580 g (0.067 mmol) of an aqueous solution of 2% by mass ethylene glycol diglycidyl ether was added as a crosslinking agent to obtain a hydrous gel polymer.

Under stirring, 0.265 g of an aqueous solution of 45% by mass diethylenetriaminepentaacetic acid pentasodium was added to the hydrous gel polymer obtained after the second-stage polymerization. Thereafter, the flask was immersed in an oil bath set to 125°C, and 241.6 g of water was extracted out of the system while n-heptane was refluxed by azeotropic distillation with n-heptane and water. Thereafter, 4.42 g (0.507 mmol) of an aqueous solution of 2% by mass ethylene glycol diglycidyl ether as a surface crosslinking agent was added into the flask, and maintained at 83°C for 2 hours.

Thereafter, n-heptane was evaporated at 125°C, and the residue was dried to obtain a dried product (polymer particles). This dried product was passed through a sieve having an aperture of 850 $\mu$m, and 0.2% by mass of amorphous silica (Oriental Silicas Corporation, Tokusil NP-S) was mixed therewith. Thereby, 228.2 g of water-absorbent resin particles was obtained. A water retention capacity of the obtained water-absorbent resin particles for a physiological saline solution was 43 g/g.

(Comparative Example 1) A cylindrical round-bottomed separable flask was prepared, which had an inner diameter of 11 cm and a capacity of 2 L, and was equipped with a reflux condenser, a dropping funnel, a nitrogen gas introduction tube, and as a stirrer, a stirring blade having four inclined paddle blades, each having a blade diameter of 5 cm, in a two-tier manner. 293 g of n-heptane as a hydrocarbon dispersion medium was weighed into this flask, and 0.736 g of a maleic anhydride-modified ethylene-propylene copolymer (HI-WAX 1105A, manufactured by Mitsui Chemicals, Inc.) as a polymeric dispersant were added thereinto. The reaction solution was heated to 80°C while being stirred to dissolve the dispersant, and then was cooled to 50°C.

Meanwhile, 92.0 g (1.03 moles) of an aqueous solution of 80.5% by mass acrylic acid as an ethylenically unsaturated monomer was weighed into a beaker with an internal capacity of 300 mL, and 147.7 g of an aqueous solution of 20.9% by mass sodium hydroxide was added dropwise thereto while cooling the beaker from the outside to perform neutralization to 75 mol%. Thereafter, 0.092 g of hydroxylethyl cellulose (HEC AW-15F, manufactured by Sumitomo Seika Chemicals Co., Ltd.) as a thickener, 0.092 g (0.339 mmol) of 2,2'-azobis(2-amidinopropane) dihydrochloride and 0.018 g (0.068 mmol) of potassium persulfate as a radical polymerization initiator, and 0.0046 g (0.026 mmol) of ethylene glycol diglycidyl ether as an internal crosslinking agent were added and dissolved to prepare a first-stage aqueous liquid.

The prepared aqueous liquid was added into the separable flask and stirred for 10 minutes. Thereafter, a surfactant solution, which was obtained by dissolving 0.736 g of sucrose stearic acid ester with HLB 3 (RYOTO Sugar Ester S-370, manufactured by Mitsubishi-Chemical Foods Corporation) as a surfactant in 6.62 g of n-heptane through heating, was further added into the flask. While stirring the reaction solution at 550 rpm as a rotational speed of the stirrer, the inside of the system was sufficiently replaced with nitrogen. Thereafter, the flask was immersed in a water bath at 70°C to raise its temperature, and polymerization was performed for 60 minutes. Thereby, a first-stage polymerization slurry liquid was obtained.

Meanwhile, 128.8 g (1.43 moles) of an aqueous solution of 80.5% by mass acrylic acid as an ethylenically unsaturated monomer was weighed into another beaker with an internal capacity of 500 mL, and 159.0 g of an aqueous solution of 27% by mass sodium hydroxide was added dropwise thereto while cooling the beaker from the outside to perform neutralization to 75 mol%. Thereafter, 0.129 g (0.475 mmol) of 2,2'-azobis(2-amidinopropane) dihydrochloride and 0.026 g (0.095 mmol) of potassium persulfate as a radical polymerization initiator, and 0.0116 g (0.067 mmol) of ethylene glycol diglycidyl ether as an internal crosslinking agent were added and dissolved to prepare a second-stage aqueous liquid.

While stirring the aqueous liquid at 1,000 rpm as a rotational speed of the stirrer, the inside of the separable flask system was cooled to 25°C, and then a total amount of the second-stage aqueous liquid was added to the first-stage polymerization slurry liquid. The inside of the system was replaced with nitrogen for 30 minutes. Thereafter, the flask was immersed in a water bath again at 70°C to raise its temperature, and a polymerization reaction was performed for 60 minutes.

Under stirring, 0.265 g of an aqueous solution of 45% by mass diethylenetriaminepentaacetic acid pentasodium was added to the hydrous gel polymer obtained after the second-stage polymerization. Thereafter, the flask was immersed in an oil bath set to 125°C, and 233.5 g of water was extracted out of the system while n-heptane was refluxed by azeotropic distillation with n-heptane and water. Thereafter, 4.42 g (0.507 mmol) of an aqueous solution of 2% by mass ethylene glycol diglycidyl ether as a surface crosslinking agent was added into the flask, and maintained at 83°C for 2 hours.

Thereafter, n-heptane was evaporated at 125°C, and the residue was dried to obtain a dried product (polymer particles). This dried product was passed through a sieve having an aperture of 850 μm, and 0.2% by mass of amorphous silica (Oriental Silicas Corporation, Tokusil NP-S) was mixed therewith. Thereby, 229.6 g of water-absorbent resin particles was obtained. A water retention capacity of the obtained water-absorbent resin particles for a physiological saline solution was 44 g/g.

(Comparative Example 2) A cylindrical round-bottomed separable flask was prepared, which had an inner diameter of 11 cm and a capacity of 2 L, and was equipped with a reflux condenser, a dropping funnel, a nitrogen gas introduction tube, and as a stirrer, a stirring blade having four inclined paddle blades, each having a blade diameter of 5 cm, in a two-tier manner. 293 g of n-heptane as a hydrocarbon dispersion medium was weighed into this flask, and 0.736 g of a maleic anhydride-modified ethylene-propylene copolymer (HI-WAX 1105A, manufactured by Mitsui Chemicals, Inc.) as a polymeric dispersant were added thereinto. The reaction solution was heated to 80°C while being stirred to dissolve the dispersant, and then was cooled to 50°C.

Meanwhile, 92.0 g (1.03 moles) of an aqueous solution of 80.5% by mass acrylic acid as an ethylenically unsaturated monomer was weighed into a beaker with an internal capacity of 300 mL, and 147.7 g of an aqueous solution of 20.9% by mass sodium hydroxide was added dropwise thereto while cooling the beaker from the outside to perform neutralization to 75 mol%. Thereafter, 0.092 g of hydroxylethyl cellulose (HEC AW-15F, manufactured by Sumitomo Seika Chemicals Co., Ltd.) as a thickener, 0.092 g (0.339 mmol) of 2,2'-azobis(2-amidinopropane) dihydrochloride and 0.018 g (0.068 mmol) of potassium persulfate as a radical polymerization initiator, and 0.0046 g (0.026 mmol) of ethylene glycol diglycidyl ether as an internal crosslinking agent were added and dissolved to prepare a first-stage aqueous liquid.

The prepared aqueous liquid was added into the separable flask and stirred for 10 minutes. Thereafter, a surfactant solution, which was obtained by dissolving 0.736 g of sucrose stearic acid ester with HLB 3 (RYOTO Sugar Ester S-370, manufactured by Mitsubishi-Chemical Foods Corporation) as a surfactant in 6.62 g of n-heptane through heating, was further added into the flask. While stirring the reaction solution at 550 rpm as a rotational speed of the stirrer, the inside of the system was sufficiently replaced with nitrogen. Thereafter, the flask was immersed in a water bath at 70°C to raise its temperature, and polymerization was performed for 60 minutes. Thereby, a first-stage polymerization slurry liquid was obtained.

Meanwhile, 128.8 g (1.43 moles) of an aqueous solution of 80.5% by mass acrylic acid as an ethylenically unsaturated monomer was weighed into another beaker with an internal capacity of 500 mL, and 159.0 g of an aqueous solution of 27% by mass sodium hydroxide was added dropwise thereto while cooling the beaker from the outside to perform neutralization to 75 mol%. Thereafter, 0.129 g (0.475 mmol) of 2,2'-azobis(2-amidinopropane) dihydrochloride and 0.026 g (0.095 mmol) of potassium persulfate as a radical polymerization initiator, and 0.0116 g (0.067 mmol) of ethylene glycol diglycidyl ether as an internal crosslinking agent were added and dissolved to prepare a second-stage aqueous liquid.

While stirring the aqueous liquid at 1,000 rpm as a rotational speed of the stirrer, the inside of the separable flask system was cooled to 25°C, and then a total amount of the second-stage aqueous liquid was added to the first-stage polymerization slurry liquid. The inside of the system was replaced with nitrogen for 30 minutes. Thereafter, the flask was immersed in a water bath again at 70°C to raise its temperature, and a polymerization reaction was performed for 60 minutes.

Under stirring, 0.265 g of an aqueous solution of 45% by mass diethylenetriaminepentaacetic acid pentasodium was added to the hydrous gel polymer obtained after the second-stage polymerization. Thereafter, the flask was immersed in an oil bath set to 125°C, and 244.4 g of water was extracted out of the system while n-heptane was refluxed by azeotropic distillation with n-heptane and water. Thereafter, 4.42 g (0.507 mmol) of an aqueous solution of 2% by mass ethylene glycol diglycidyl ether as a surface crosslinking agent was added into the flask, and maintained at 83°C for 2 hours.

Thereafter, n-heptane was evaporated at 125°C, and the residue was dried to obtain a dried product (polymer particles). This dried product was passed through a sieve having an aperture of 850 μm, and 0.2% by mass of amorphous silica (Oriental Silicas Corporation, Tokusil NP-S) was mixed therewith. Thereby, 229.6 g of water-absorbent resin particles was obtained. A water retention capacity of the obtained water-absorbent resin particles for a physiological saline solution was 51 g/g.

[Pulverization of water-absorbent resin particles] 4 g of the obtained water-absorbent resin particles was pulverized for 15 seconds by a small pulverizer (Wonder Blender WB-1) attached with a fine pulverizing lid (model number: PN-W03), and thereby pulverized particles were obtained. The above operation was repeated until a predetermined amount of pulverized particles was obtained.

The obtained water-absorbent resin particles and pulverized particles were evaluated for a median particle size, a particle size distribution, a water retention capacity for a physiological saline solution, a dissolved content, and stickiness after pulverization by the following method. The results are shown in Table 1.

[Median particle size and particle size distribution] For the water-absorbent resin particles before pulverization, JIS standard sieves were combined in the following order from the top: a sieve having an aperture of 600 $\mu$m, a sieve having an aperture of 500 $\mu$m, a sieve having an aperture of 425 $\mu$m, a sieve having an aperture of 300 $\mu$m, a sieve having an aperture of 250 $\mu$m, a sieve having an aperture of 180 $\mu$m, a sieve having an aperture of 150 $\mu$m, and a receiving tray.

For the pulverized particles, JIS standard sieves were combined in the following order from the top: a sieve having an aperture of 425 $\mu$m, a sieve having an aperture of 300 $\mu$m, a sieve having an aperture of 212 $\mu$m, a sieve having an aperture of 150 $\mu$m, a sieve having an aperture of 106 $\mu$m, a sieve having an aperture of 75 $\mu$m, a sieve having an aperture of 45 $\mu$m, and a receiving tray.

50 g of the water-absorbent resin particles or 20 g of the pulverized particles was fed to the topmost sieve among the combination of the sieves, shaken for 10 minutes using a Ro-Tap shaker, and thereby classified. After the classification, a mass of the particles remaining on each of the sieves was calculated as a mass percentage with respect to a total amount to determine a particle size distribution. By integrating values on the sieves in descending order of the particle sizes with regard to the particle size distribution, a relationship between the aperture of the sieve and the integrated value of mass percentages of the particles remaining on the sieve was plotted on a log-probability paper. The plotted points on the probability paper were connected with straight lines, and a particle size corresponding to 50% by mass of the integrated mass percentage was taken as a median particle size.

In addition, masses of the particles that had passed through the sieve having an aperture of 300 $\mu$m were integrated, and a proportion of the particles having a particle size of 300 $\mu$m or less with respect to a total amount of the particles was obtained as a particle distribution.

[Dissolved content] 500 g of an aqueous solution of 0.9% by mass NaCl was put into a 500 mL beaker. A stirring bar (8 mm$\varphi$ × 30 mm without a ring) was put into the beaker, and a rotational speed thereof was adjusted so that it rotated at 600 rpm. 2.000 g of the water-absorbent resin particles or pulverized particles was put into the above beaker, stirred at 25°C for 3 hours, and then filtered through a 75 $\mu$m standard sieve, and the filtrate was recovered. The filtrate was further suction-filtered using a Kiriyama-type funnel (filter paper: ADVANTEC, No. 6). 80 g of the obtained filtrate was weighed into a 100 mL beaker that had been constant at 140°C in advance, and was dried with a hot air dryer (manufactured by ADVANTEC, FV-320) at 140°C for 15 hours, and a mass Wa (g) of a solid content of the filtrate was measured. The above operation was carried out in the same manner without using the particles, Wb (g) of a solid content of a filtrate was measured, and a dissolved content was calculated by the following formula.

$$\text{Dissolved content (\% by mass)} = [((Wa - Wb)/80) \times 500/2] \times 100$$

[Amount of adhesion of gel (stickiness)] The measurement was performed in the environment of a temperature of 25°C and a humidity of 60 ± 10%. 50 g of an aqueous solution of 0.9% by mass sodium chloride at 25 ± 1°C was put into a 100 mL beaker, and 1.0 g of the water-absorbent resin particles was put thereinto while stirring with a stirring bar (8 mm$\varphi$ × 30 mm, without a ring) at a rotational speed of 600 rpm. The particles were swollen, and thereby a swollen gel was obtained. A filter paper 1 (ADVANTEC, No. 51A, 15 × 15 cm) was placed on a tray, an acrylic resin guide frame having an opening of 10 cm × 10 cm was placed on the filter paper 1, and a total amount of the swollen gel in the beaker was evenly dispersed in the frame. A pre-weighed filter paper 2 (ADVANTEC, No. 51A, 9.8 cm × 9.8 cm) was placed on the dispersed swollen gel. Furthermore, 19 sheets of filter paper of the same size (ADVANTEC, No. 51A, 9.8 cm × 9.8 cm) were placed on the filter paper 2 in an overlapped manner to absorb excess water of the swollen gel. A 1.0 kg weight (9.8 cm × 9.8 cm) was placed on the filter paper along the inside of the guide frame. After 1 minute, the weight was removed, and then the guide frame was removed. The 19 sheets of filter paper placed on the top in an overlapped manner were removed, the filter paper 2 was slowly peeled off from the swollen gel, and a mass of the filter paper 2 to which a part of the swollen gel adhered was measured. By subtracting a mass (g) of the filter paper 2 before the load from a mass of the filter paper 2 after the load, a mass of the swollen gel adhered to the filter paper 2 was calculated and used as an index for evaluating stickiness. That is, as an amount of the swollen gel adhered to the filter paper 2 becomes larger, stickiness is likely to be generated, and as an amount of the swollen gel adhered to the filter paper 2 becomes smaller, stickiness is unlikely to be generated.

The water-absorbent resin particles of the examples having a low dissolved content after pulverization had reduced stickiness as compared with the comparative examples.

[Water retention capacity for physiological saline solution] A cotton bag (cotton broadcloth No. 60, 100 mm in width × 200 mm in length) into which 2.0 g of the water-absorbent resin particles had been weighed was placed in a beaker having a capacity of 500 mL. 500 g of an aqueous solution of 0.9% by mass sodium chloride (physiological saline solution) was poured into the cotton bag containing the water-absorbent resin particles at once so that a lump could not be produced. The upper part of the cotton bag was bound with a rubber band and left to stand for 30 minutes, and thereby the water-absorbent resin particles were swollen. The cotton bag after an elapse of 30 minutes was dehydrated for 1 minute using a dehydrator (manufactured by KOKUSAN Co., Ltd., product number: H-122) which had been set at a centrifugal force of 167 G, and a mass Wc (g) of the dehydrated cotton bag containing the swollen gel was measured. By performing the same operation without addition of the water-absorbent resin particles, a mass Wd (g) of an empty cotton bag upon moisturizing was measured, and a water retention capacity for a physiological saline solution was calculated by the following formula.

$$\text{Water retention capacity for physiological saline solution (g/g)}$$
$$= [\text{Wc} - \text{Wd}]/2.00$$

[Table 1]

| | Before pulverization | | | After pulverization | | | Amount of gel adhered to filter paper (g) |
|---|---|---|---|---|---|---|---|
| | Proportion of particles with 300 μm or less (% by mass) | Median particle size (μm) | Dissolved content (% by mass) | Proportion of particles with 300 μm or less (% by mass) | Median particle size (μm) | Dissolved content (% by mass) | |
| Example 1 | 25 | 346 | 13.0 | 76 | 135 | 25.7 | 3.4 |
| Example 2 | 14 | 492 | 15.8 | 99 | 85 | 33.3 | 4.8 |
| Comparative Example 1 | 27 | 342 | 11.0 | 96 | 113 | 40.5 | 10.4 |
| Comparative Example 2 | 20 | 355 | 22.9 | 87 | 162 | 43.1 | 12.4 |

**Reference Signs List**

10 absorbent, 10a water-absorbent resin particle, 10b fiber layer, 20a, 20b core wrap, 30 liquid-permeable sheet, 40 liquid-impermeable sheet, 100 absorbent article

**Claims**

**1.** Water-absorbent resin particles comprising:

a crosslinked polymer having a structural unit derived from an ethylenically unsaturated monomer including at least one compound selected from the group consisting of (meth)acrylic acid and a salt thereof, wherein a proportion of (meth)acrylic acid and a salt thereof is 70 to 100 mol% with respect to a total amount of monomer units in the crosslinked polymer, and a dissolved content is 10% by mass or more and 40% by mass or less, and a dissolved content when the water-absorbent resin particles are pulverized so that a median particle size is 80 to 165 μm is 15% by mass or more and 40% by mass or less, where the dissolved contents are measured by the following dissolved content measurement method which is performed as follows:

500 g of an aqueous solution of 0.9% by mass NaCl is put into a 500 mL beaker and stirred at 600 rpm, 2 g of the water-absorbent resin particles or pulverized particles thereof is put into the beaker, stirred at 25°C for 3 hours, and then filtered through a 75 μm standard sieve, and a filtrate is recovered, the obtained filtrate is further suction-filtered using a type 6 filter paper defined in JIS P 3801, 80 g of the filtrate obtained by suction filtration is weighed into a pre-weighed 100 mL beaker and dried with a hot air dryer at 140°C for 15 hours, and a mass Wa (g) of a solid content of the filtrate is measured, and the above operation is carried out in the same manner without using the particles, Wb (g) of a solid content of a filtrate is measured, and a dissolved content is calculated by the following formula,

$$\text{dissolved content (\% by mass)} = [((Wa - Wb)/80) \times 500/2] \times 100.$$

2. The water-absorbent resin particles according to claim 1, wherein a median particle size is 250 to 600 μm.

3. The water-absorbent resin particles according to claim 1 or 2, wherein a proportion of particles having a particle size of 300 μm or less is 55% by mass or less with respect to a total amount of the water-absorbent resin particles.

4. The water-absorbent resin particles according to any one of claims 1 to 3, wherein the dissolved content when the water-absorbent resin particles are pulverized so that a median particle size is 80 to 165 μm is a value when the water-absorbent resin particles are pulverized so that a proportion of particles having a particle size of 300 μm or less are 70% by mass or more with respect to a total amount of the water-absorbent resin particles.

5. The water-absorbent resin particles according to any one of claims 1 to 4, wherein a water retention capacity for a physiological saline solution is 20 to 70 g/g.

6. An absorbent comprising the water-absorbent resin particles according to any one of claims 1 to 5.

7. An absorbent article comprising the absorbent according to claim 6.

8. The absorbent article according to claim 7, wherein the article is a diaper.

EP 3 896 106 A1

# Fig.1

## INTERNATIONAL SEARCH REPORT

| International application No. |
| --- |
| PCT/JP2019/048799 |

### A. CLASSIFICATION OF SUBJECT MATTER

C08F 265/02(2006.01)i; C08J 3/12(2006.01)i; C08J 3/24(2006.01)i; C08L 101/12(2006.01)i; C08F 2/44(2006.01)i; A61F 13/53(2006.01)i; B01J 20/26(2006.01)i; B01J 20/28(2006.01)i
FI: C08J3/12 Z CEY; C08L101/12; C08F2/44 C; C08F265/02; C08J3/24; A61F13/53 300; B01J20/28 Z; B01J20/26 D

According to International Patent Classification (IPC) or to both national classification and IPC

### B. FIELDS SEARCHED

Minimum documentation searched (classification system followed by classification symbols)
C08J3/00-C08J3/28; C08F265/02; C08L101/12; C08F2/44; A61F13/53; B01J20/26; B01J20/28

Documentation searched other than minimum documentation to the extent that such documents are included in the fields searched
Published examined utility model applications of Japan 1922-1996
Published unexamined utility model applications of Japan 1971-2020
Registered utility model specifications of Japan 1996-2020
Published registered utility model applications of Japan 1994-2020

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
| --- | --- | --- |
| A | JP 2003-088552 A (SUMITOMO SEIKA CHEMICALS CO., LTD.) 25.03.2003 (2003-03-25) entire text | 1-8 |
| A | WO 2018/092864 A1 (NIPPON SHOKUBAI CO., LTD.) 24.05.2018 (2018-05-24) entire text | 1-8 |
| A | JP 2006-057075 A (NIPPON SHOKUBAI CO., LTD.) 02.03.2006 (2006-03-02) entire text | 1-8 |
| A | WO 2016/158976 A1 (NIPPON SHOKUBAI CO., LTD.) 06.10.2016 (2016-10-06) entire text | 1-8 |
| A | JP 2015-110801 A (SUMITOMO SEIKA CHEMICALS CO., LTD.) 18.06.2015 (2015-06-18) entire text | 1-8 |
| A | JP 2015-142909 A (ASAHI KASEI CHEMICALS CORP.) 06.08.2015 (2015-08-06) entire text | 1-8 |

☒ Further documents are listed in the continuation of Box C.    ☒ See patent family annex.

| | |
| --- | --- |
| * Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
| --- | --- |
| 28 February 2020 (28.02.2020) | 10 March 2020 (10.03.2020) |

| Name and mailing address of the ISA/<br>Japan Patent Office<br>3-4-3, Kasumigaseki, Chiyoda-ku,<br>Tokyo 100-8915, Japan | Authorized officer |
| --- | --- |
| | Telephone No. |

Form PCT/ISA/210 (second sheet) (January 2015)

**EP 3 896 106 A1**

<div align="center">

**INTERNATIONAL SEARCH REPORT**

</div>

| International application No. |
| --- |
| PCT/JP2019/048799 |

**C (Continuation).   DOCUMENTS CONSIDERED TO BE RELEVANT**

| Category* | Citation of document, with indication, where appropriate, of the relevant passages | Relevant to claim No. |
| --- | --- | --- |
| A | WO 2015/053372 A1 (NIPPON SHOKUBAI CO., LTD.) 16.04.2015 (2015-04-16) entire text | 1-8 |
| A | WO 2015/152299 A1 (NIPPON SHOKUBAI CO., LTD.) 08.10.2015 (2015-10-08) entire text | 1-8 |
| A | WO 2014/041968 A1 (NIPPON SHOKUBAI CO., LTD.) 20.03.2014 (2014-03-20) entire text | 1-8 |

Form PCT/ISA/210 (continuation of second sheet) (January 2015)

20

**INTERNATIONAL SEARCH REPORT**
Information on patent family members

International application No.

PCT/JP2019/048799

| Patent Documents referred in the Report | Publication Date | Patent Family | Publication Date |
|---|---|---|---|
| JP 2003-088552 A | 25 Mar. 2003 | (Family: none) | |
| WO 2018/092864 A1 | 24 May 2018 | US 2019/0329219 A1 entire text EP 3543279 A1 entire text CN 109996835 A entire text KR 10-2019-0077541 A entire text | |
| JP 2006-057075 A | 02 Mar. 2006 | WO 2005/092955 A1 entire text US 2007/0141338 A1 entire text EP 1730218 A1 entire text CN 1934170 A entire text KR 10-2007-0004521 A entire text | |
| WO 2016/158976 A1 | 06 Oct. 2016 | US 2018/0094131 A1 entire text EP 3278873 A1 entire text CN 107405600 A entire text KR 10-2017-0132800 A entire text | |
| JP 2015-110801 A | 18 Jun. 2015 | (Family: none) | |
| JP 2015-142909 A | 06 Aug. 2015 | (Family: none) | |
| WO 2015/053372 A1 | 16 Apr. 2015 | EP 3056268 A1 entire text CN 105658323 A entire text KR 10-2016-0068768 A entire text | |
| WO 2015/152299 A1 | 08 Oct. 2015 | US 2017/0136441 A1 entire text EP 3127606 A1 entire text CN 106132534 A entire text KR 10-2016-0140659 A entire text | |
| WO 2014/041968 A1 | 20 Mar. 2014 | US 2015/0225514 A1 entire text EP 2896645 A1 entire text CN 104619755 A entire text KR 10-2015-0056572 A entire text | |

Form PCT/ISA/210 (patent family annex) (January 2015)

**EP 3 896 106 A1**

## REFERENCES CITED IN THE DESCRIPTION

**Patent documents cited in the description**

- JP H6218007 A